# EUROPÄISCHE PATENTSCHRIFT

(11) **EP 0 485 736 B1**
(45) Veröffentlichungstag und Bekanntmachung des Hinweises auf die Patenterteilung: **03.05.1995**
(21) Anmeldenummer: 91117108.0
(22) Anmeldetag: 08.10.1991
(51) Int. Cl.: A61F 13/00

(54) **Medizinisches Saugtuch**
Absorbent medical tampon
Tampon médical absorbant

(30) Priorität: 14.11.1990 DE 4036208
(43) Veröffentlichungstag der Anmeldung: 20.05.1992
(73) Patentinhaber: VOSTRA MEDICALPRODUKTE VERTRIEBS- UND BERATUNGSGESELLSCHAFT mbH, D-52022 Aachen (DE)
(72) Erfinder: Harren, Bernd, W-5100 Aachen (DE); Moll, Philipp, W-5100 Aachen (DE)
(74) Vertreter: Selting, Günther, Dipl.-Ing.

(56) Entgegenhaltungen:
- EP-A- 0 120 710
- EP-A- 0 242 415
- FR-A- 2 430 222
- FR-A- 2 525 245
- FR-A- 2 597 123

## Beschreibung

Die Erfindung betrifft ein medizinisches Saugtuch sowie ein Verfahren zum Herstellen eines derartigen medizinischen Saugtuchs.

Medizinische Saugtücher werden im operativen Bereich verwendet, um Blut und andere Körperflüssigkeit eines Patienten aufzusaugen, beispielsweise um aus Operationsöffnungen des Patientenkörpers Flüssigkeit zu entfernen, um den Zugang zu freiliegenden Körperorganen und die Einsehbarkeit während der Operation zu verbessern. In Operationsräumen steht normalerweise ein Vorrat von derartigen Saugtüchern zur Verfügung, die auch als Bauchtücher bezeichnet werden. Sofern solche Saugtücher Einmalartikel sind, die nach Gebrauch fortgeworfen werden, stellen sie wegen der möglichen bakteriellen Verseuchung Sondermüll dar, dessen Entsorgung mit erheblichen Schwierigkeiten verbunden ist. Bekannt sind auch wiederverwendbare Saugtücher, die aus mehreren Lagen Gewebe bestehen, das an den Kanten vernäht ist, um ein Ausfransen zu vermeiden. Normalerweise bestehen solche Saugtücher aus einem Sack, dessen Wände jeweils aus mehreren Gewebelagen bestehen. Durch das Vernähen der eingeschlagenen Schnittkanten erreicht das Saugtuch in den Randbereichen ein Mehrfaches der ursprünglichen Lagenanzahl. Solche Saugtücher können zwar nach Gebrauch in Spezialverfahren gewaschen und desinfiziert werden, jedoch ist ihre hygienische Unbedenklichkeit in den Randbereichen und Ecken umstritten. In den zahlreichen aufeinanderliegenden Gewebelagen an den Rändern und in den Ecken können sich Blut und andere Körperflüssigkeiten festsetzen, die in das Saugtuch eindringen und beim Waschen nicht mit der erforderlichen Sicherheit entfernt werden. Solche Flüssigkeitsreste bilden, insbesondere wenn sie eingetrocknet sind, organische Schmutzherde, die ggf. nicht den Reinheitsanforderungen des Deutschen Arzneibuches entsprechen.

Aus der dem Oberbegriff des Anspruchs 1 zugrundeliegenden DE-A-23 40 180 ist ein medizinisches Saugtuch bekannt, bei dem drei der vier Kantenbereiche durch Versiegeln gegen Ausfasern gesichert sind, während der vierte Kantenbereich von einer Saumkante gebildet wird. Zwischen den Kanten verlaufen in der Fläche des Saugtuchs Bindenähte, in denen die Gewebelagen durch Bindenähte untereinander verbunden sind, welche in der Weise hergestellt sind, daß ein Webmuster entsteht, bei dem die Bindegarne sich über mehrere Gewebelagen des Saugtuchs erstrecken, so daß die Gewebelagen im Mittelbereich untereinander verwebt sind. Durch das Versiegeln der Kantenbereiche werden diese zwar gegen Ausfasern gesichert, jedoch werden sie gleichzeitig hart, was für ein medizinisches Saugtuch, das im Operationsbereich verwendet wird, sehr nachteilig ist.

Der Erfindung liegt die Aufgabe zugrunde, ein medizinisches Saugtuch zu schaffen, bei dem die Kantenbereiche und die Eckbereiche keine Nester für das Festsetzen von Kontaminationen bilden u. somit das Saugtuch wiederverwendbar machen.

Die Lösung dieser Aufgabe erfolgt erfindungsgemäß mit den im Patentanspruch 1 angegebenen Merkmalen.

Bei dem erfindungsgemäßen medizinischen Saugtuch sind die einzelnen saugfähigen Lagen mindestens an den Kantenbereichen untereinander verwebt. Dies bedeutet, daß Webfäden der einen Lage in eine benachbarte Lage übergehen. Aus diesem Grund ist die Stärke des Saugtuch im Kantenbereich nicht größer als im Mittenbereich. Sie ist im Gegenteil im Kantenbereich sogar geringer, so daß keine schwer auswaschbaren Wulste entstehen. Die in den Kantenbereichen längs- und querlaufenden Webfäden bestehen aus hydrophobem Material, das Flüssigkeit abstößt, während das medizinische Saugtuch insgesamt aus hydrophilem Material besteht. Die Kantenbereiche nehmen am Saugverhalten des Saugtuchs nicht teil. Damit wird zwar die Saugfähigkeit des Saugtuchs insgesamt geringfügig verringert, jedoch bleibt die Weichheit auch an den Kantenbereichen erhalten.

Die hydrophoben Webfäden sollten vorzugsweise aus thermoplastischem Kunststoff bestehen. Ein Vorteil dieser Maßnahme besteht darin, daß der thermoplastische Kunststoff seine hydrophoben Eigenschaften auch nach häufigem Waschen nicht verliert. Ferner besteht die Möglichkeit, die Kunststoffäden in den Kantenbereichen durch Wärmebehandlung und Druck mit gleichartigen Fäden oder mit saugfähigen Fäden zu verbinden und somit die Tuchkanten zu verfestigen. Dadurch wird das Ausfransen oder Ausfasern des Tuchs an den Kanten verhindert.

Das erfindungsgemäße medizinische Saugtuch hat in der Regel eine Größe von 40 x 40 cm und es besteht vorzugsweise aus mindestens drei miteinander verwebten Gewebelagen. Es ist nicht erforderlich, daß die Gewebelagen ausschließlich in den Kantenbereichen miteinander verwebt sind. Vielmehr können punkt- oder linienförmige Durchverbindungen der Gewebelagen auch innerhalb der von den Kantenbereichen umschlossenen Felder vorgesehen sein, um die Gewebelagen dort miteinander zu verheften und ein Auseinanderspreizen der Gewebelagen zu vermeiden.

Bei dem erfindungsgemäßen Verfahren zum Herstellen eines medizinischen Saugtuchs soll entweder in Gebrauchsbreite oder ein Großtuch gewebt werden, das der Größe zahlreicher Saugtücher entspricht. Im zweiten Fall werden dann in den Grenzbereichen benachbarter Saugtücher die Gewebebahnen miteinander verwebt, wobei die längs der Kanten verlaufenden Webfäden aus hydrophobem Material gewählt werden können. Anschließend erfolgt das Abtrennen der Saugtücher entlang der aneinandergrenzenden Kantenbereiche. Das Großtuch kann als endlose Bahn gewebt werden.

Im folgenden wird unter Bezugnahme auf die Zeichnungen ein Ausführungsbeispiel der Erfindung näher erläutert.

Es zeigen:
- Fig. 1: eine Darstellung eines Großtuchs, von dem einzelne Saugtücher abgetrennt werden,
- Fig. 2: im vergrößerten Maßstab ein Querschnitt entlang der Linie II-II von Fig. 1 und
- Fig. 3: ein aus dem Großtuch von Fig. 1 abgetrenntes Saugtuch.

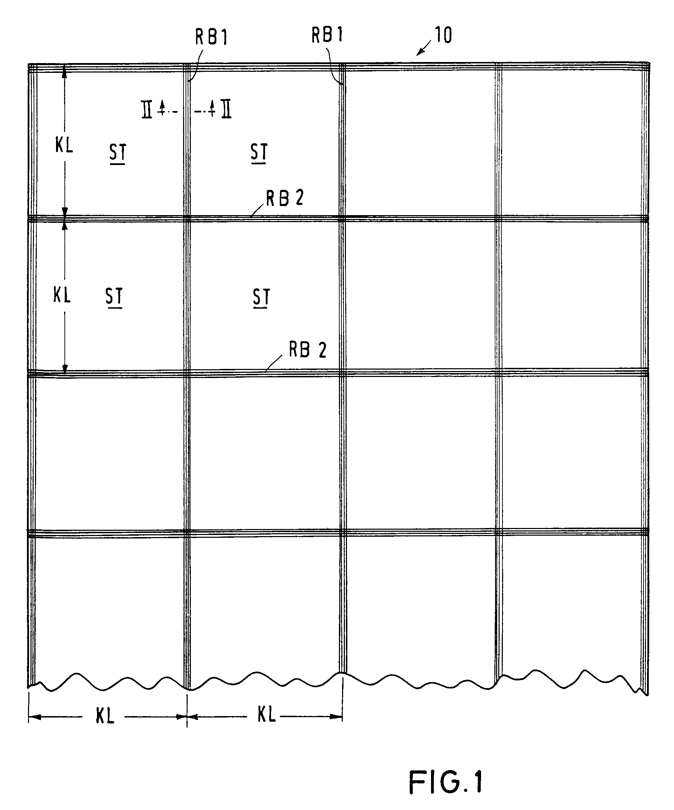

In Fig. 1 ist ein Großtuch 10 dargestellt, das als Ganzes durch Weben hergestellt wird und das mehrere später abzutrennende Saugtücher ST umfaßt. Die Saugtücher ST sind in dem Großtuch 10 nach Art eines Schachbrettmusters enthalten. Sie bestehen aus rechteckigen Gebilden mit einer Kantenlänge KL von beispielsweise 40 x 40 cm. Das Großtuch 10 ist durch streifenförmige Randbereiche RB1 und RB2 in Längsrichtung und in Querrichtung unterteilt, wobei jeder dieser Randbereiche später die beiden Kantenbereiche zweier benachbarter Saugtücher ST bildet. Das Großtuch 10 wird als Materialbahn kontinuierlich auf einer Webmaschine hergestellt, indem mehrere Gewebelagen gewebt werden.

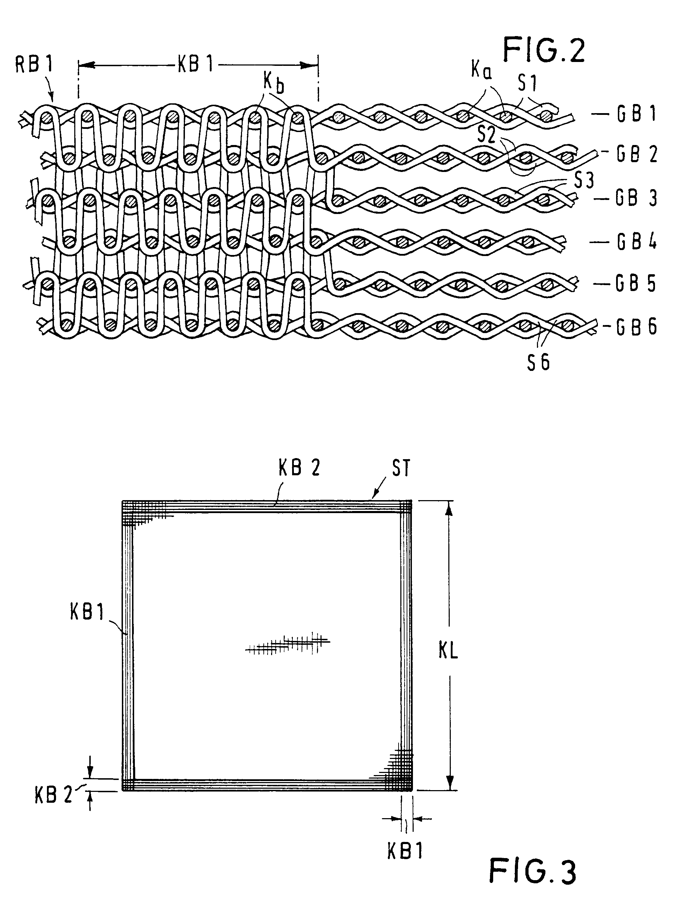

In Fig. 2 sind insgesamt sechs Gewebelagen GB1-GB6 dargestellt, die jeweils aus Kettfäden Kₐ und K_{b} sowie aus Schußfäden bestehen. Die Schußfäden der einzelnen Gewebelagen sind mit S1-S6 bezeichnet.

Die Gewebelagen GB1-GB6 sind, mit Ausnahme der Kantenbereiche des Saugtuchs, voneinander getrennt und sie verlaufen parallel zueinander. In den Kantenbereichen KB1 und KB2 des Saugtuchs ST sind die Gewebelagen miteinander verwebt. Die längslaufenden Kantenbereiche des Saugtuchs sind mit KB1 und die querlaufenden Kantenbereiche mit KB2 bezeichnet (Fig. 3).

Wie aus Fig. 2 zu ersehen ist, sind in dem längslaufenden Kantenbereich KB1 in der obersten Gewebelage GB1 Kettfäden K_{b} und Schußfäden S1, sich in üblicher Weise kreuzend, vorgesehen. Die Kettfäden Kₐ sind im Mittelbereich des Saugtuchs, also in der Saugtuchfläche, mit Ausnahme der Kantenbereiche, aus saugfähigem Material hergestellt, vorzugsweise aus Baumwollmaterial. Diejenigen Kettfäden K_{b}, die sich im Randbereich RB1 bzw. im Kantenbereich KB1 befinden, bestehen dagegen aus hydrophobem Material, insbesondere aus thermoplastischem Kunststoff.

Die Schußfäden sämtlicher Gewebelagen bestehen aus saugfähigem Material (vorzugsweise Baumwolle), mit Ausnahme derjenigen Schußfäden, die in den querlaufenden Kantenbereichen KB2 angeordnet sind. Diese können ebenfalls aus hydrophobem Material bestehen.

Die Schußfäden S2 der zweiten Gewebelage GB2 bilden mit den zugehörigen Kettfäden Kₐ ein übliches Gewebe. In den Kantenbereichen KB1 sind jedoch die Schußfäden S2 nicht nur mit den Kettfäden derselben Gewebelage verwebt sondern darüber hinaus mit den Kettfäden KB der darüberliegenden Gewebelage GB1. Die Schußfäden S2 sind im Randbereich RB1 also mit den Kettfäden K_{b} zweier benachbarter Gewebelagen GB1 und GB2 verwebt.

In gleicher Weise sind in jedem Randbereich RB1 jeweils die Schußfäden einer Gewebelage (z.B. GB5) mit den Kettfäden K_{b} der darüberliegenden Gewebelage (z.B. GB4) verwebt.

In den querlaufenden Randbereichen RB2 sind die Schußfäden über die gesamte Breite des Großtuches zusätzlich mit den Kettfäden der darüberliegenden Gewebebahn verwebt, so daß in den querlaufenden Randbereichen RB2 alle Gewebebahnen über die Gesamtbreite der Großbahn miteinander verwebt sind. In den querlaufenden Randbereichen RB2 bestehen die Kettfäden vorzugsweise aus hydrophobem Material, z.B. aus thermoplastischem Kunststoff.

Nachdem das Großtuch 10 gemäß Fig. 1 auf die beschriebene Weise gewebt worden ist, können aus dem Großtuch die einzelnen Saugtücher ST geschnitten werden. Dabei wird der Trennschnitt jeweils mittig durch die längslaufenden Randbereiche RB1 und durch die querlaufenden Randbereiche RB2 geführt. Da jeder dieser Randbereiche die doppelte Breite eines Kantenbereichs hat, haben zwei aneinandergrenzende Saugtücher jeweils einen längslaufenden Kantenbereich KB1 und einen querlaufenden Kantenbereich KB2.

Vor dem Abtrennen der Saugtücher oder während des Abtrennens können die Randbereiche RB1 und RB2 durch entsprechende beheizte Preßwerkzeuge zusammengedrückt werden, so daß die thermoplastischen Fäden sich in den Randbereichen über sämtliche Lagen hinweg miteinander verschweißen bzw. mit den saugfähigen Fasern verbinden. Dadurch wird das Tuchmaterial in den Kantenbereichen KB1 und KB2 verfestigt, so daß nicht die Gefahr der Flusenbildung oder des Ausfransens besteht. Vorzugsweise erfolgt das thermische Verbinden bzw. Verfestigen in den Randbereichen dadurch, daß zwei Messerbalken mit Schweißbalken kombiniert sind, so daß beim Gegeneinanderbewegen der Messerbalken das Abtrennen der Saugtücher und gleichzeitig die Verfestigung der Kantenbereiche durchgeführt wird. Ergänzend ist es ebenfalls vorstellbar, durch Ultraschallschneiden und -verschweissen oder aber Ultraschallverschweißen plus rotierende Messer den oben geschilderten Trenneffekt zu erzielen.

In den Eckbereichen, in denen sich zwei Kantenbereiche KB1 und KB2 überlagern, können sämtliche Kett- und Schußfäden aus hydrophobem Material bestehen. Durch die thermoplastische Verschmelzung in den Kantenbereichen werden die Randfäden gegen Ablösen und Vorstehen fixiert.

Das Großtuch 10 kann auch aus einem Streifen von der Breite eines einzigen Saugtuches bestehen, der zahlreiche Saugtücher hintereinander enthält.

Die Saugtücher können nach ihrer Benutzung gewaschen und desinfiziert und anschließend wiederverwendet werden. Nach Beendigung der Lebensdauer eines Saugtuchs ist eine Aufbereitung zum Zwecke des Recyclings möglich, wobei die Kantenbereiche KB1 und KB2 abgeschnitten werden, wenn sie aus thermoplastischem Material bestehen. Da der Innenbereich vorzugsweise ausschließlich aus Baumwollmaterial besteht, kann er wie üblicher Baumwoll-Abfall aufbereitet und weiterverarbeitet werden, das gleiche gilt für andere nicht thermoplastische Gewebe.

## Patentansprüche

1. Medizinisches Saugtuch mit mehreren saugfähigen Gewebelagen (GB1-GB6), die in den Kantenbereichen (KB1,KB2) miteinander verbunden sind,
**dadurch gekennzeichnet,**
daß die Verbindung in den Kantenbereichen (KB1, KB2) eine Webverbindung ist, wobei die in den Kantenbereichen längs der Kanten verlaufenden Webfäden aus hydrophobem Material bestehen, während die übrigen Webfäden aus hydrophilem Material bestehen.

2. Medizinisches Saugtuch nach Anspruch 1, dadurch gekennzeichnet, daß die hydrophoben Webfäden aus thermoplastischem Kunststoff bestehen und mit den quer zu dem jeweiligen Kantenbereich verlaufenden Fäden durch Schweißung verbunden sind.

3. Medizinisches Saugtuch nach Anspruch 1 oder 3, dadurch gekennzeichnet, daß mindestens vier Gewebelagen vorhanden sind.

4. Verfahren zum Herstellen eines medizinischen Saugtuchs aus mehreren saugfähigen Gewebelagen,
**dadurch gekennzeichnet,**
daß ein Großtuch (10) gewebt wird, das der Größe zahlreicher Saugtücher (ST) entspricht und bei dem mehrere Gewebelagen (GB1-GB6) längs der aneinandergrenzenden Kantenbereiche der herzustellenden Saugtücher miteinander verwebt werden, wobei die in den Kantenbereichen (KB1,KB2) längs der Kanten verlaufenden Webfäden aus hydrophobem Material gewählt werden, während die übrigen Webfäden aus hydrophilem Material bestehen, und daß von dem Großtuch (10) die einzelnen Saugtücher (ST) durch Trennung entlang der aneinandergrenzenden Kantenbereiche abgetrennt werden.

5. Verfahren nach Anspruch 4, dadurch gekennzeichnet, daß als hydrophobe Webfäden thermoplastische Webfäden benutzt werden, die untereinander und mit den anderen Webfäden durch Druck und Wärme verbunden werden.

6. Verfahren nach Anspruch 5, dadurch gekennzeichnet, daß das Verbinden durch Druck und Wärme gleichzeitig mit dem Abtrennen der Saugtücher (ST) erfolgt.

## Claims

1. An absorbent medical tampon comprising a plurality of absorptive tissue layers (GB1-GB6) being connected to each other in their edge regions (KB1, KB2),
**characterized in**
that said connection in the edge regions (KB1,KB2) is a woven connection, wherein those weaving threads which in the edge regions extend along the edges are made from hydrophobic material, whereas the other weaving threads are made from hydrophilic material.

2. The absorbent medical tampon according to claim 1, characterized in that the hydrophobic weaving threads are made from thermoplastic synthetic material and are connected by welding to the threads extending transversely to the respective edge region.

3. The absorbent medical tampon according to claim 1 or 2, characterized in that at least four tissue layers are provided.

4. A method for manufacturing an absorbent medical tampon from a plurality of absorptive tissue layers,
**characterized by**
weaving a large-sized tampon (10) which corresponds to the size of a large number of absorbent tampons (ST) and in which a plurality of tissue layers (GB1-GB6) are interwoven with each other along the adjacent edge regions of the absorbent tampons to be manufactured, wherein those weaving threads which in the edge regions (KB1,KB2) extend along the edges are selected from hydrophobic materials and the other weaving threads are made from hydrophilic materials, and characterized by detaching the individual absorbent tampons (ST) from the large-sized tampon (10) by separation along the adjacent edge regions.

5. The method according to claim 4, characterized in that, for said hydrophilic weaving threads, there are used thermoplastic weaving threads connected to each other and to the other weaving threads through heat and pressure.

6. The method according to claim 5, characterized in that said heat and pressure connection is performed simultaneously with the detaching of the absorbent tampons (ST).

## Revendications

1. Tampon médical absorbant avec plusieurs couches de tissu absorbant (GB1-GB6) qui sont assemblées l'une à l'autre dans les zones de bord (KB1, KB2), caractérisé par le fait que l'assemblage dans les zones de bord (KB1, KB2) est un assemblage tissé, les fils de tissage qui s'étendent dans les zones de bord, le long des bords, étant en une matière hydrophobe, tandis que les autres fils de tissage sont en une matière hydrophile.

2. Tampon médical absorbant selon la revendication 1, caractérisé par le fait que les fils de tissage hydrophobes sont en une matière synthétique thermoplastique et sont assemblés par soudage aux fils s'étendant transversalement par rapport à la zone de bord concernée.

3. Tampon médical absorbant selon la revendication 1 ou 2, caractérisé par le fait qu'au moins quatre couches de tissu sont présentes.

4. Procédé pour la fabrication d'un tampon médical absorbant constitué de plusieurs couches de tissu absorbant, caractérisé par le fait que l'on tisse un grand tampon (10) qui correspond à la grandeur d'une pluralité de tampons absorbants (ST) et dans lequel plusieurs couches de tissu (GB1-GB6) sont tissées l'une avec l'autre le long des zones de bord adjacentes des tampons absorbants à fabriquer, les fils de tissage s'étendant, dans les zones de bord (KB1, KB2), le long des bords étant choisis en une matière hydrophobe, tandis que les autres fils de tissage sont en une matière hydrophile, et que les différents tampons absorbants (ST) sont séparés du grand tampon (10) par séparation le long des zones de bord adjacentes l'une à l'autre.

5. Procédé selon la revendication 4, caractérisé par le fait qu'on utilise, comme fils de tissages hydrophobes, des fils de tissage thermoplastiques qui sont assemblés entre eux et aux autres fils de tissage par pression et chaleur.

6. Procédé selon la revendication 5, caractérisé par le fait que l'assemblage par pression et chaleur se fait en même temps que la séparation des tampons absorbants (ST).
